# EUROPEAN PATENT APPLICATION

(11) **EP 4 775 233 A1**
(43) Date of publication of application: **15.07.2026**
(21) Application number: 24875653.8
(22) Date of filing: 14.09.2024
(51) Int. Cl.: A61M 1/00, A61B 17/22

(54) **NEGATIVE PRESSURE SUCTION SHEATH**

(71) Applicant: Innovex Medical Co., Ltd., Shanghai 201201 (CN)
(72) Inventor: YAN, Hang, Shanghai 201201 (CN); LIU, Luobin, Shanghai 201201 (CN); ZHENG, Zhongwei, Shanghai 201201 (CN)
(74) Representative: Argyma
(86) International application number: PCT/CN2024/119192
(87) International publication number: WO 2026/055983

(57) **Abstract**

The present invention provides a negative pressure suction sheath including a sheath tube, a negative pressure joint, and a dilator, the negative pressure joint includes a main channel cavity and a negative pressure suction channel cavity located at one side of the main channel cavity, a proximal end of the sheath tube is connected to a distal end of the main channel cavity, and the dilator is inserted from a proximal end of the main channel cavity and extends out of a distal end of the sheath tube, and is detachably connected to the main channel cavity; one side of the main channel cavity is provided with a side channel port, and the negative pressure suction channel cavity is communicated with the side channel port; a cavity wall of the main channel cavity is further provided with a pressure regulating port, the pressure regulating port is disposed corresponding to the side channel port or disposed at one end, away from the sheath tube, of the side channel port, and a negative pressure in the sheath tube may be adjusted by adjusting an opening size of the pressure regulating port. Therefore, an operator does not need to leave to adjust a negative pressure device to adjust a suction force in the sheath tube, but only needs to adjust the size of the pressure regulating port on the hand-held negative pressure joint, so as to adjust the negative pressure in the sheath tube.

## Description

### TECHNICAL FIELD

The present invention relates to the field of medical device technologies, and in particular, to a negative pressure suction sheath.

### BACKGROUND

Renal calculi have plagued mankind for a long time. Complications caused by calculi in urethra usually require surgical intervention. In traditional surgeries for removing calculi, surgeons need to cut open abdomens of patients to remove the calculi, and thus the patients need to stay in hospitals for a long time for recovering.

The use of minimally invasive lithotomy devices has shortened time for patients to suffer pain and recover. Because of a unique anatomical structure of the kidney, it is difficult to reach calyces, especially lower renal calyces, during endoscopic combined intrarenal surgeries. Unfortunately, it is still very difficult to remove fragmented calculi from the calyces even with intraoperative access to the calyces, removal of calculi, and effective lithotripsy. As a result, the patients have to expel fragmented calculi by themselves, which will prolong the treatment time and experience of suffering pain and discomfort.

Ureteral access sheaths (UAS) are used in urological surgeries. A ureteral access sheath is first placed in a ureter prior to a clinical urological surgery, so as to establish a surgical channel. During the surgery, an endoscope, a laser fiber, a lithotomy device, and the like, are introduced through the surgical channel to remove some small calculi from the ureteral access sheath.

An existing ureteral access sheath usually includes a sheath, a negative pressure joint, a dilator, and the like. The sheath is connected to a negative pressure device through the negative pressure joint, so as to suck and discharge intraoperative fragmented calculi and fluids by a negative pressure. Because a suction force in the sheath tube needs to be adjusted according to the intrapelvic pressure (IPP) during a surgery, and the negative pressure device is usually disposed in a place where an operator (i.e., a surgeon) cannot reach with his/her hands, for example, disposed on a wall of an operating room, the operator cannot leave to adjust the negative pressure device to adjust the suction force in the sheath during the surgery.

Therefore, how to adjust the suction force in the sheath according to the intrapelvic pressure during the surgery has become a technical problem to be urgently resolved in the art.

### SUMMARY

To resolve the foregoing technical problem, the present invention provides a negative pressure suction sheath including a sheath, a negative pressure joint, and a dilator, where the negative pressure joint includes a main channel cavity and a negative pressure suction channel cavity located at one side of the main channel cavity, a proximal end of the sheath is connected to a distal end of the main channel cavity, and the dilator is inserted from a proximal end of the main channel cavity and extends out of a distal end of the sheath, and is detachably connected to the main channel cavity; one side of the main channel cavity is provided with a side channel port, and the negative pressure suction channel cavity is communicated with the side channel port; a cavity wall of the main channel cavity is further provided with a pressure regulating port, the pressure regulating port is disposed corresponding to the side channel port or disposed at one end, away from the sheath, of the side channel port, and a negative pressure in the sheath may be adjusted by adjusting an opening size of the pressure regulating port.

Optionally, the pressure regulating port is located at one side opposite to the side channel port.

Optionally, the pressure regulating port is located at one side perpendicular to the side channel port.

Optionally, the pressure regulating port is provided with a regulating mechanism capable of adjusting the opening size thereof.

Optionally, the regulating mechanism is a valve or a slider, the valve or the slider is movably disposed on the pressure regulating port, and the opening size of the pressure regulating port is adjusted by pushing the valve or the slider.

Optionally, the proximal end of the main channel cavity is further provided with a sealing washer and a fixing member, and the sealing washer is hermetically fixed at the proximal end of the main channel cavity by using the fixing member; the sealing washer is provided with an inner hole, so that the dilator or/and a surgical device inserted into the main channel cavity is fixedly sleeved in the inner hole of the sealing washer to achieve a fixed connection with the main channel cavity.

Optionally, in a natural state, a diameter of the inner hole of the sealing washer is not larger than an outer diameter of the dilator or/and the surgical device.

Optionally, in a natural state, a diameter of the inner hole of the sealing washer is larger than an outer diameter of the dilator or/and the surgical device, a degree of compression on the sealing washer is adjusted by using the fixing member, and then a size of the inner hole of the sealing washer is adjusted, so that the dilator or/and the surgical device inserted into the main channel cavity is fixedly sleeved in the inner hole of the sealing washer.

Optionally, the fixing member is a threaded fixing member, the threaded fixing member fixes the sealing washer at the proximal end of the main channel cavity through a threaded connection with the main channel cavity, and the degree of compression on the sealing washer may be adjusted by loosening or tightening the threaded fixing member.

Optionally, the sheath is a single-cavity or multi-cavity structure, and correspondingly, the sealing washer has one or more inner holes.

Optionally, the surgical device includes an endoscope.

Optionally, a tip end of the sheath is a flexible structure.

Optionally, the sheath is a single-layer tube or a three-layer reinforced tube, if the sheath is a single-layer tube, the sheath is made of a polymer material; or if the sheath is a three-layer reinforced tube, an exterior layer thereof is made of a thermoplastic material, a middle layer thereof is a coiled spring structure or a metal braided wire structure, and an inner layer thereof is a polymer tube with a low friction coefficient.

Compared with the conventional technology, technical solutions of embodiments of the present invention have the following beneficial effects.

According to the present invention, the pressure regulating port is disposed on the main channel cavity of the negative pressure joint. Therefore, the operator does not need to leave to adjust the negative pressure device to adjust the suction force in the sheath, but only needs to adjust the opening size of the pressure regulating port on the hand-held negative pressure joint, so as to adjust the negative pressure in the sheath.

Further, according to the present invention, the pressure regulating port is disposed at a corresponding side channel port of the main channel cavity or disposed on a cavity wall at one end, away from the sheath, of the side channel port rather than being disposed on the sheath, the negative pressure suction channel cavity, and a cavity wall, at the distal end of the side channel port, of the main channel cavity, which resolves the technical problems of accumulation of calculi in the negative pressure suction channel cavity and blockage of the negative pressure suction channel cavity caused by disposing the pressure regulating port on the negative pressure suction channel cavity, thereby improving a negative pressure suction effect.

### BRIEF DESCRIPTION OF DRAWINGS

In order to explain the technical solutions in the embodiments of the present invention or in the conventional technology more clearly, the drawings used in the description of the embodiments or the conventional technology will be briefly introduced below. Clearly, the drawings in the following description are merely some embodiments of the present invention. For those of ordinary skill in the art, other drawings can be obtained based on these drawings without creative efforts.
FIG. 1 is a schematic diagram of a structure of a negative pressure suction sheath according to an embodiment of the present invention;
FIG. 2 is a schematic diagram of a structure of a negative pressure joint according to an embodiment of the present invention (in a closed state of a pressure regulating port);
FIG. 3 is a schematic diagram of a structure of a negative pressure joint according to an embodiment of the present invention (in an open state of a pressure regulating port);
FIG. 4 is a schematic diagram of a structure of a negative pressure suction sheath according to another embodiment of the present invention;
FIG. 5 is a comparison diagram of a pressure regulating port located at one side opposite to a side channel port and located at one side perpendicular to the side channel port in the present invention;
FIG. 6 is a schematic diagram of a mounting structure of a sealing washer according to an embodiment of the present invention;
FIG. 7 is a comparison diagram of a sealing washer in a natural state and in an axially compressed state according to an embodiment of the present invention; and
FIG. 8 is a schematic diagram of a structure of a W-shaped negative pressure joint according to an embodiment of the present invention.

### DESCRIPTION OF EMBODIMENTS

As described in the background, an existing ureteral access sheath usually includes a sheath, a negative pressure joint, a dilator, and the like. The sheath is connected to a negative pressure device through the negative pressure joint, so as to suck and discharge intraoperative fragmented calculi and fluids by a negative pressure. Because a suction force in the sheath needs to be adjusted according to the intrapelvic pressure(IPP) during a surgery, and the negative pressure device is usually disposed in a place where an operator (i.e., a surgeon) cannot reach with his/her hands, for example, disposed on a wall of an operating room, the operator cannot leave to adjust the negative pressure device to adjust the suction force in the sheath during the surgery.

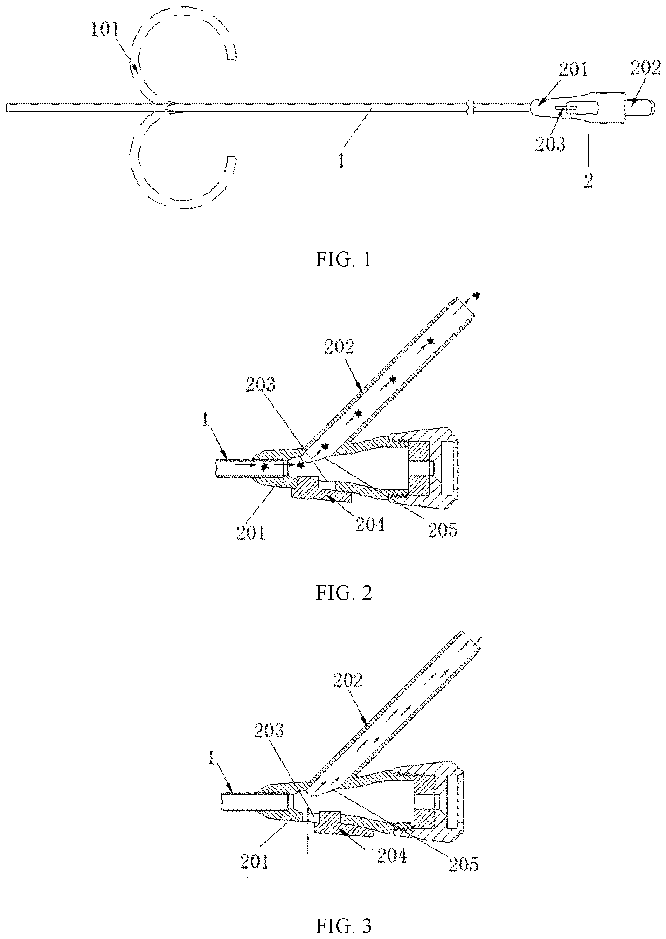

To resolve the foregoing technical problem, references can be made to FIG. 1 to FIG. 7. The present invention provides a negative pressure suction sheath including a sheath 1, a negative pressure joint 2, and a dilator 3, where the negative pressure joint 2 includes a main channel cavity 201 and a negative pressure suction channel cavity 202 located at one side of the main channel cavity 201, a proximal end of the sheath 1 is connected to a distal end of the main channel cavity 201, and the dilator 3 is inserted from a proximal end of the main channel cavity 201 and extends out of a distal end of the sheath 1, and is detachably connected to the main channel cavity 201; one side of the main channel cavity 201 is provided with a side channel port 205, and the negative pressure suction channel cavity 202 is communicated with the side channel port 205; a cavity wall of the main channel cavity 201 is further provided with a pressure regulating port 203, the pressure regulating port 203 is disposed corresponding to the side channel port 205 or disposed at one end, away from the sheath 1, of the side channel port 205, and a negative pressure in the sheath 1 may be adjusted by adjusting an opening size of the pressure regulating port 203.

According to the present invention, the pressure regulating port 203 is disposed on the main channel cavity 201 of the negative pressure joint 2. Therefore, the operator does not need to leave to adjust the negative pressure device to adjust the suction force in the sheath 1, but only needs to adjust the size of the pressure regulating port 203 on the hand-held negative pressure joint 2, so as to adjust the negative pressure in the sheath 1.

A specific method for adjusting the negative pressure in the sheath 1 is as follows: The pressure regulating port 203 is kept fully open or minimally blocked when a minimum negative pressure is needed. When more negative pressure is needed, the pressure regulating port 203 is fully closed to a maximum extent.

In the present invention, there is no specific restriction on a way for adjusting the size of the pressure regulating port 203, for example, the size of the pressure regulating port 203 may be adjusted by pressing the pressure regulating port 203 with a finger; or a regulating mechanism 204 may be disposed on the pressure regulating port 203, and the size of the pressure regulating port 203 may be adjusted by manually adjusting the regulating mechanism 204.

In the art, the negative pressure joint 2 is usually of a Y-shaped structure, which includes a main channel cavity 201 and a negative pressure suction channel cavity 202 located on one side of the main channel cavity 201. The negative pressure suction channel cavity 202 is connected to a negative pressure device, so that a negative pressure suction airway is formed between the negative pressure suction channel cavity 202 and the sheath 1, and fragmented calculi in the human body are sucked out of the human body through the negative pressure suction airway. Because the negative pressure suction channel cavity 202 is located on the negative pressure suction airway and is connected to the negative pressure device, a person skilled in the art could have easily conceived that a pressure regulating port is disposed on a cavity wall of the negative pressure suction channel cavity 202, and a negative pressure is adjusted by adjusting the size of the pressure regulating port. However, this way of disposing the pressure regulating port on the negative pressure suction channel cavity 202 destroys integrity of the cavity wall of the negative pressure suction channel cavity 202. Therefore, when the pressure regulating port is fully opened or incompletely opened, fragmented calculi are easily discharged from the pressure regulating port when they are discharged outward through the negative pressure suction channel cavity 202, and fragmented calculi are easily stuck at the pressure regulating port when they are not smaller than an opening caliber of the pressure regulating port or a plurality of fragmented calculi are crowded and discharged from the pressure regulating port. Therefore, these fragmented calculi easily prevent other fragmented calculi from being discharged from the negative pressure suction channel cavity 202. Over time, more and more fragmented calculi accumulate in the negative pressure suction channel cavity 202, blocking the negative pressure suction channel cavity 202. As a result, fragmented calculi in the body can no longer be discharged from the negative pressure suction channel cavity 202.

To resolve the foregoing technical problem, according to the present invention, the pressure regulating port 203 is disposed at a corresponding side channel port 205 of the main channel cavity 201 or disposed on a cavity wall at one end, away from the sheath 1, of the side channel port 205 rather than being disposed on the sheath 1, the negative pressure suction channel cavity 202, and a cavity wall, at the distal end of the side channel port 205, of the main channel cavity 201, which resolves the technical problems of accumulation of calculi in the negative pressure suction channel cavity and blockage of the negative pressure suction channel cavity caused by disposing the pressure regulating port on the negative pressure suction channel cavity, thereby improving a negative pressure suction effect.

The technical solutions in the embodiments of the present invention will be clearly and fully described in combination with the accompanying drawings of the embodiments of the present invention. Clearly, the described embodiments are merely some rather than all of the embodiments of the present invention. Based on the embodiments of the present invention, all other embodiments obtained by those of ordinary skill in the art without creative efforts should fall within the protection scope of the present invention.

One embodiment of the present invention provides a negative pressure suction sheath, including a sheath 1, a negative pressure joint 2, and a dilator 3, where the negative pressure joint 2 includes a main channel cavity 201 and a negative pressure suction channel cavity 202 located at one side of the main channel cavity 201, a proximal end of the sheath 1 is connected to a distal end of the main channel cavity 201, and the dilator 3 is inserted from a proximal end of the main channel cavity 201 and extends out of a distal end of the sheath 1, and is detachably connected to the main channel cavity 201; one side of the main channel cavity 201 is provided with a side channel port 205, and the negative pressure suction channel cavity 202 is communicated with the side channel port 205.

In the present invention, there is no specific restriction on an included angle between the negative pressure suction channel cavity 202 and the main channel cavity 201. The negative pressure suction channel cavity 202 extends outward from an outer surface of the main channel cavity 201, and forms an angle in the range of greater than 0° to less than 180° with the proximal main channel cavity 201. In one embodiment, the negative pressure suction channel cavity 202 extends outward from the outer surface of the main channel cavity 201 and towards a proximal direction of the main channel cavity 201, that is, an acute angle, such as 25° to 45°, is formed between the negative pressure suction channel cavity 202 and a proximal portion of the main channel cavity 201, so as to effectively discharge calculi or other foreign matters.

In the present invention, there is no specific restriction on whether an axis of the negative pressure suction channel cavity 202 intersects with an axis of the main channel cavity 201. As one embodiment, the axis of the negative pressure suction channel cavity 202 intersects with the axis of the main channel cavity 201.

As one embodiment, a cavity wall of the main channel cavity 201 is provided with a pressure regulating port 203, the pressure regulating port 203 is disposed corresponding to the side channel port 205, that is, the pressure regulating port 203 is located at one side opposite to or beside the side channel port 205.

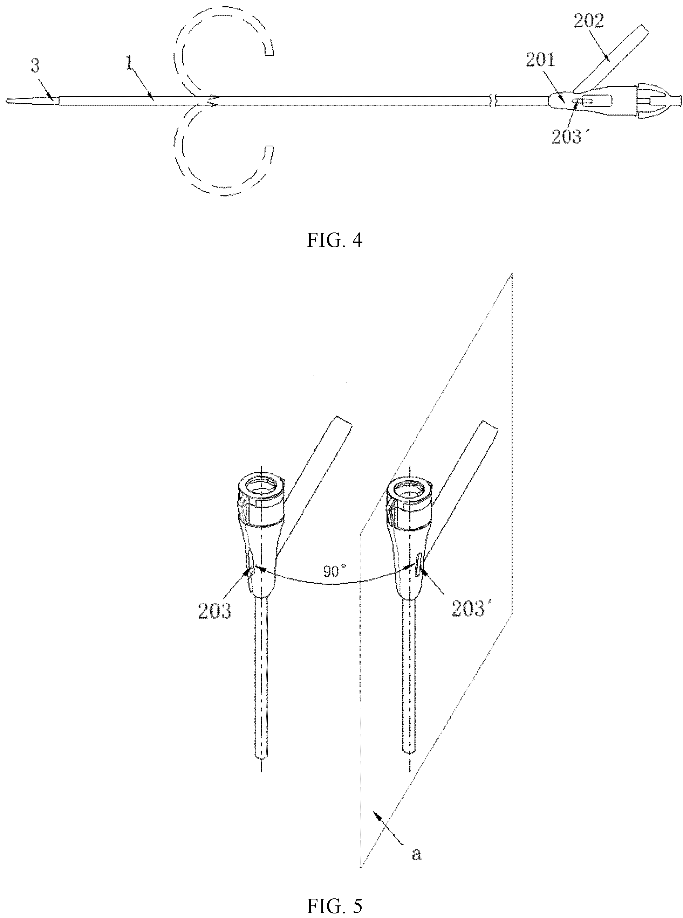

References can be made to FIG. 1 to FIG. 3, and a left side view of FIG. 5. The left side view of FIG. 5 shows that a pressure regulating port 203 is located at one side opposite to the side channel port 205. As one specific implementation, the pressure regulating port 203 is located at the side opposite to the side channel port 205, that is, the cavity wall of the main channel cavity 201 is provided with the pressure regulating port 203 at the side opposite to the side channel port 205. Therefore, the side channel port 205 and the pressure regulating port 203 easily form a "draught" gas convection, and a negative pressure suction effect may be enhanced by using the principle of convection enhancement of "draught". At the moment when the pressure regulating port 203 is closed, a negative pressure suction force may be rapidly increased to efficiently suck out fragmented calculi, thereby improving suction efficiency. If the negative pressure suction force is small, the fragmented calculi can be sucked out after suction for many times, thereby increasing suction time, and thus increasing pain of a patient.

References can be made to FIG. 4 and a right side view of FIG. 5. The right side view of FIG. 5 shows that a pressure regulating port 203' is located at one side perpendicular to the side channel port 205. As a second specific implementation, the pressure regulating port 203' is located at the side perpendicular to the side channel port 205, that is, a perpendicular line from the pressure regulating port 203' to the axis of the main channel cavity 201 is perpendicular to a perpendicular line from the side channel port 205 to the axis of the main channel cavity 201, or in other words, the pressure regulating port 203' is located at a perpendicular line of a plane A on which the axis of the main channel cavity 201 and the axis of the negative pressure suction channel cavity 202 are located. From the perspective of ergonomics, when a hand holding the suction sheath holds the negative pressure suction channel cavity 202, a thumb used to adjust an opening size of the pressure regulating port 203' is closer to the perpendicular side, which makes it easier for the operator ( surgeon) to operate and relieves fatigue of the operator.

As a third specific implementation, the pressure regulating port 203 is located at the end, away from the sheath tube 1, of the side channel port 205. Therefore, the pressure regulating port 203 bypasses the negative pressure suction airway formed between the negative pressure suction channel cavity 202 and the sheath 1, which resolves the technical problems of accumulation of calculi in the negative pressure suction channel cavity 202 and blockage of the negative pressure suction channel cavity 202 caused by disposing the pressure regulating port 203 on the negative pressure suction channel cavity 202, thereby improving a negative pressure suction effect.

In the conventional technology, when negative pressure suction is needed, it is required to press a finger at the pressure regulating port 203 all the time to maintain a sealed state, and long-term operation will lead to fatigue of the operator. In addition, surgical operation may also be affected. Taking the right hand of the operator as a dominant hand for example, in existing flexible endoscopy, an operator needs to hold an endoscope with his/her right hand, and fix a ureteral access sheath(UAS) with his/her left hand. Once a finger of the left hand presses a pressure regulating port, the ureteral access sheath cannot be fixed certainly, which will lead to movement of the ureteral access sheath and affect lithotomy with the endoscope. To resolve this technical problem, in the present invention, the pressure regulating port 203 is provided with a regulating mechanism 204 capable of adjusting the opening size. The operator may move the regulating mechanism 204 to a desired position with his/her thumb while holding the suction sheath with his/her left hand, without pressing the pressure regulating port 203 with his/her finger all the time.

In one embodiment, the regulating mechanism 204 is a valve or a slider, the valve or the slider is movably disposed on the pressure regulating port 203, and the opening size of the pressure regulating port 203 is adjusted by pushing the valve or the slider.

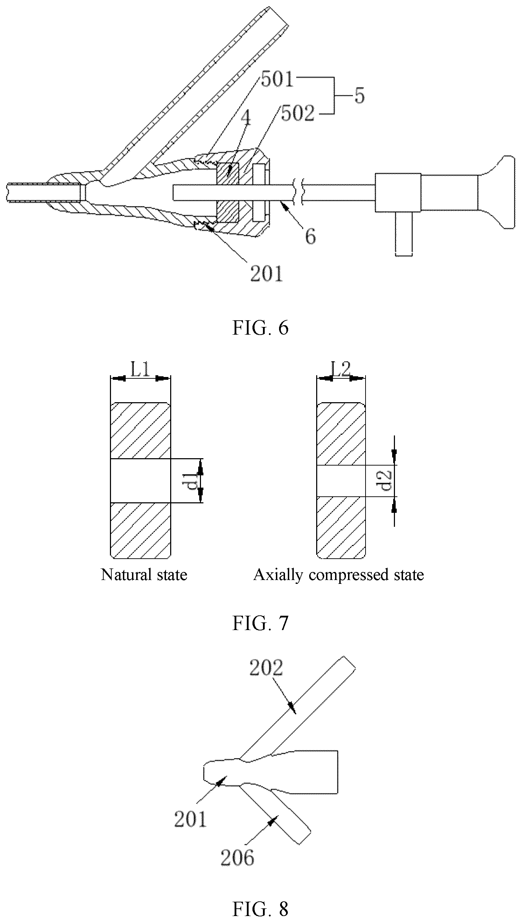

References can be made to FIG. 6. In one embodiment, the proximal end of the main channel cavity 201 is further provided with a sealing washer 4 and a fixing member, and the sealing washer 4 is hermetically fixed at the proximal end of the main channel cavity 201 by using the fixing member, so that the proximal end of the main channel cavity 201 is sealed, thereby ensuring negative pressure sealability. The sealing washer 4 is provided with an inner hole, so that the dilator 3 or/and a surgical device inserted into the main channel cavity 201 is fixedly sleeved in the inner hole of the sealing washer 4 to achieve a fixed connection with the main channel cavity 201.

In this embodiment, there is no restriction on a way for fixed connection between the fixing member and the proximal end of the main channel cavity 201, which may be a detachable or non-detachable fixing way such as clamping or threaded connection. To facilitate replacement of the sealing washer 4, preferably, the fixing member and the main channel cavity 201 are fixedly connected in a detachable way.

In a natural state, when a diameter of the inner hole of the sealing washer 4 is not larger than an outer diameter of the dilator 3 or/and the surgical device, the dilator 3 or/and the surgical device may be directly inserted into the inner hole of the sealing washer 4 to be fixedly sleeved in the sealing washer 4.

In a natural state, when a diameter of the inner hole of the sealing washer 4 is larger than an outer diameter of the dilator 3 or/and the surgical device, a degree of compression on the sealing washer 4 needs to be adjusted by using the fixing member, and then a size of the inner hole of the sealing washer 4 is adjusted, so that the dilator 3 or/and the surgical device inserted into the main channel cavity 201 is fixedly sleeved in the inner hole of the sealing washer 4.

In the foregoing fixedly sleeved state, the sealing washer 4 is sleeved over the dilator 3 or/and the surgical device through the inner hole, and there is no gap between the sealing washer 4 and a periphery of the dilator 3 or/and the surgical device.

As one implementation, the fixing member is a threaded fixing member 5, the threaded fixing member 5 fixes the sealing washer 4 at the proximal end of the main channel cavity 201 through a threaded connection with the main channel cavity 201; the sealing washer 4 is provided with an inner hole, a degree of compression on the sealing washer 4 is adjusted by loosening or tightening the threaded fixing member 5, and then a size of the inner hole of the sealing washer 4 is adjusted, so that the dilator 3 or/and the surgical device inserted into the main channel cavity 201 is fixedly sleeved in the inner hole of the sealing washer 4 to achieve a fixed connection with the main channel cavity 201.

In the natural state, a thickness of the sealing washer 4 is L1, and when a diameter d1 of the inner hole of the sealing washer 4 is not smaller than the outer diameter of the dilator 3 or/and the surgical device, the dilator 3 or/and the surgical device may pass smoothly through the inner hole of the sealing washer 4 into the sheath tube 1. By tightening the threaded fixing member 5, threads thereof axially compress the sealing washer 4, and after the sealing washer 4 is deformed under axial compression, a thickness thereof is L2 (L1 > L2); and a diameter of the inner hole of the sealing washer 4 is reduced to d2 (d2 < d1). Therefore, the sealing washer 4 holds the dilator 3 or/and the surgical device tightly through the inner hole, which can not only control an axial position of the dilator 3 or/and the surgical device to prevent displacement, but also form a closed state to ensure negative pressure sealability. On the contrary, the diameter of the inner hole is restored to the natural state by loosening the threaded fixing member 5 to release the sealing washer 4 from an axially compressed state, so that the dilator 3 or/and the surgical device may be moved.

In the natural state, when the diameter of the inner hole of the sealing washer 4 is smaller than the outer diameter of the dilator 3 or/and the surgical device, the dilator 3 or/and the surgical device may be fixedly sleeved with the sealing washer 4 without compressing the sealing washer 4.

In this embodiment, there is no restriction on a specific type of the surgical device, which may be an endoscope 6. When the dilator 3 is inserted into the sheath 1, the proximal end of the dilator 3 is located in the inner hole of the sealing washer 4, and the fixed connection between the dilator 3 and the negative pressure joint 2 is achieved by tightening the threaded fixing member 5. After the sheath 1 is inserted into the human body together with the dilator 3 fixed to the negative pressure joint 2, the threaded fixing member 5 is loosened, the dilator 3 is pulled out, the endoscope 6 is inserted into the sheath 1 through the inner hole of the sealing washer 4, and the threaded fixing member 5 is tightened to achieve a fixed connection between the endoscope and the negative pressure joint 2. When the endoscope 6 needs to be moved, the threaded fixing member 5 may be loosened to move the endoscope 6 for diagnosis and treatment. Because of a variable diameter of the inner hole of the sealing washer 4, the sealing washer may be applied to dilators 3 and endoscopes 6 of different sizes.

In this embodiment, when fragmented calculi are small, they may enter a gap between the endoscope and the sheath 1, and eventually be discharged through the negative pressure suction channel cavity 202. When fragmented calculi are large but still small enough to enter the sheath 1, the endoscope may be retracted to a position that is just close to the distal end of the negative pressure suction channel cavity 202, allowing the fragmented calculi to be extracted successively from the sheath 1 and the negative pressure suction channel cavity 202.

In this embodiment, there is no restriction on a quantity of inner holes of the sealing washer 4, and the quantity may be set according to an inner cavity structure of the sheath 1. If the sheath 1 is a single-cavity structure, the sealing washer 4 has one inner hole; or if the sheath 1 is a multi-cavity structure, the sealing washer 4 has a plurality of inner holes.

In the present invention, there is no restriction on a specific construction of the threaded fixing member 5 for fixing the sealing washer 4 at the proximal end of the main channel cavity 201. As one embodiment, the threaded fixing member 5 includes a sleeve structure 501, and the sleeve structure 501 is coaxially disposed with the main channel cavity 201. The proximal end of the main channel cavity 201 is provided with an external thread, the distal end of the sleeve structure 501 is open, an inner wall of the distal end of the sleeve structure 501 is provided with an internal thread adapted to the external thread, and the sleeve structure 501 is in a threaded connection with the external thread of the main channel cavity 201 through the internal thread. The proximal end of the sleeve structure 501 is provided with a cover 502, the cover 502 is provided with a through hole corresponding to the inner hole of the sealing washer 4, and the dilator 3 or/and the surgical device successively pass through the through hole of the cover 502 and the inner hole of the sealing washer 4 into the sheath tube 1. An outer diameter of the sealing washer 4 is larger than an inner diameter of the proximal end of the main channel cavity 201, and larger than the through hole of the cover 502. Therefore, when the sealing washer 4 is fixed at the proximal end of the main channel cavity 201 through the threaded fixing member 5, the cover 502 and a proximal end face of the main channel cavity 201 that are located at both sides of the sealing washer 4 will compress the sealing washer 4 at the same time. Therefore, the sealing washer 4 will also seal the through hole of the cover 502 while holding the dilator 3 or/and the surgical device tightly, so that the proximal end of the main channel cavity 201 is sealed, thereby ensuring negative pressure sealability.

In the conventional technology, an entire body of the sheath 1 is relatively hard, and cannot be bent with bending of a flexible endoscope, so it is difficult to reach some parts (such as the kidney) of the human body. Therefore, calculi in these parts cannot be cleared by negative pressure suction, and the calculi can only be captured by using a device such as a stone retrieval basket inserted into the sheath 1, thus causing pain and economic burden on patients. To resolve this technical problem, in this embodiment, a tip end 101 (i.e., the distal end) of the sheath 1 may be arranged as a flexible structure, thereby improving negative pressure lithotomy efficiency, reducing the use of devices such as stone retrieval baskets, and alleviating the economic burden on the patients. The 5-15 cm tip end of the sheath 1 has a hardness different from a posterior segment, and the tip end may be passively bent with the bending of the endoscope to reach the renal pelvis and calyces of the kidney, and directly capture fragmented calculi to suck them out of the body.

A tip of the dilator 3 is flexibly designed to reduce implantation injury. A 10-40 mm segment, including a conical part, of the head end of the dilator 3 is arranged to have a hardness different from that of a main body segment, so that when cavities and tracts of the human body are narrow and twisty, the dilator can smoothly get over tortuous places through a flexible head segment to avoid injuring mucosae or puncturing and avulsing a ureter, thereby improving implantation safety.

A main body of the dilator 3 is made of a polymer material such as polyethylene and polypropylene. The main body has a suitable hardness, and gives consideration to passability and supportability during implantation. The dilator 3 may be designed as a single-cavity structure or a multi-cavity structure. A first cavity channel is configured to pass a guide wire, and a second channel or another channel is configured to inject a fluid or pass a visual observation device and another diagnostic and therapeutic device. The dilator also includes a dilator handle, which is used for detachable connection with the negative pressure joint 2. The dilator handle is usually made of a polymer material such as polypropylene, and has a suitable hardness and some deformation resistance for mounting to and unmounting from the negative pressure joint 2.

The sheath 1 is a three-layer reinforced tube, and an exterior layer thereof is made of a thermoplastic material such as nylon, which gives consideration to good hardness and softness, and allows a tube body to have good supportability and toughness. A middle layer is a coiled spring structure or a metal braided wire structure, so that the tube body has good bending resistance and supportability, thereby ensuring stability of the channel. An inner layer is usually a Teflon tube or another polymer tube with a low friction coefficient, so that a tube cavity has good surface smoothness or passability, thereby reducing insertion and extraction resistance of an endoscope and another surgical device, and protecting the device.

The sheath 1 may be designed as a single-cavity structure or a multi-cavity structure. A first cavity channel is configured to pass the dilator 3 and an endoscopic lithotomy device, and a second cavity channel is configured to pass a safety guide wire or another diagnostic and therapeutic device.

In the present invention, there is no restriction on a specific part of the human body for which the negative pressure suction sheath is suitable to suck and discharge intraoperative fragmented calculi or/and fluids by a negative pressure. Therefore, in the present invention, there is no restriction on a size of the sheath 1, and the size may be set based on actual use needs. For example, the negative pressure suction sheath is used to treat ureteral calculi, renal calculi, biliary calculi, and the like. The sheath 1 has an inner diameter of 8-16 Fr, an outer diameter of 10-18 Fr, and an effective length of 10-60 cm. When in use, the sheath 1 is inserted into the human body, and the negative pressure joint 2 is located outside the human body.

The negative pressure joint is usually made of a transparent polymer material such as polycarbonate to facilitate visual observation of a withdrawal distance for endoscopic lithotomy, a negative pressure suction effect, and discharge of calculi. The main channel cavity 201 of the negative pressure joint 2 is designed as a thin-to-thick funnel, with an end for connecting the sheath 1 being a thin port, and an end for the endoscope to go in and out being a thick port. The end for the endoscope is arranged to be larger than the end for the sheath 1 by 2 Fr or more to improve introduceability of the endoscope device when going in and out. The negative pressure joint 2 may be a Y-shaped joint, that is, one side of the main channel cavity 201 is provided with a negative pressure suction channel cavity 202 for negative pressure suction. References can be made to FIG. 8. The negative pressure joint 2 may also be a W-shaped joint, that is, in addition to the negative pressure suction channel cavity 202, the side of the main channel cavity 201 may also be additionally provided with a side channel 206 or even a plurality of side channels 206 for injecting a contrast medium or placing a pressure/temperature measuring catheter and another device. An inner diameter of the negative pressure suction channel cavity 202 is not smaller than the inner diameter of the sheath tube 1 to ensure that suction efficiency of the negative pressure suction channel cavity 202 is not lower than that of the main channel cavity.

The negative pressure suction channel cavity 202 is communicated with a main tube, the other end of the main tube is connected to a collection container, one end of an auxiliary tube is connected to the collection container, and the other end of the auxiliary tube is connected to a negative pressure device, so that the negative pressure device may be activated to remove fragmented calculi in a tissue.

In conclusion, according to the present invention, the pressure regulating port 203 is disposed on the main channel cavity 201 of the negative pressure joint 2. Therefore, the operator does not need to leave to adjust the negative pressure device to adjust the suction force in the sheath 1, but only needs to adjust the size of the pressure regulating port 203 on the hand-held negative pressure joint 2, so as to adjust the negative pressure in the sheath 1.

Further, according to the present invention, the pressure regulating port 203 is disposed at a corresponding side channel port 205 of the main channel cavity 201 or disposed on a cavity wall at one end, away from the sheath 1, of the side channel port 205 rather than being disposed on the sheath tube 1, the negative pressure suction channel cavity 202, and a cavity wall, at the distal end of the side channel port 205, of the main channel cavity 201, which resolves the technical problems of accumulation of calculi in the negative pressure suction channel cavity 202 and blockage of the negative pressure suction channel cavity 202 caused by disposing the pressure regulating port 203 on the negative pressure suction channel cavity 202, thereby improving a negative pressure suction effect.

Although the present invention has been disclosed as above, the present invention is not limited thereto. Any person skilled in the art may make various changes and modifications without departing from the spirit and scope of the present invention. Therefore, the protection scope of the present invention shall be subjected to the scope defined by the claims.

## Claims

1. A negative pressure suction sheath, comprising a sheath (1), a negative pressure joint (2), and a dilator (3), wherein the negative pressure joint (2) comprises a main channel cavity (201) and a negative pressure suction channel cavity (202) located at one side of the main channel cavity (201), a proximal end of the sheath (1) is connected to a distal end of the main channel cavity (201), and the dilator (3) is inserted from a proximal end of the main channel cavity (201) and extends out of a distal end of the sheath (1), and is detachably connected to the main channel cavity (201); one side of the main channel cavity (201) is provided with a side channel port (205), and the negative pressure suction channel cavity (202) is communicated with the side channel port (205); a cavity wall of the main channel cavity (201) is further provided with a pressure regulating port (203), the pressure regulating port (203) is disposed corresponding to the side channel port (205) or disposed at one end, away from the sheath (1), of the side channel port (205), and a negative pressure in the sheath (1) may be adjusted by adjusting an opening size of the pressure regulating port (203).

2. The negative pressure suction sheath according to claim 1, wherein the pressure regulating port (203) is located at one side opposite to the side channel port (205).

3. The negative pressure suction sheath according to claim 1, wherein the pressure regulating port (203) is located at one side perpendicular to the side channel port (205).

4. The negative pressure suction sheath according to claim 1, wherein the pressure regulating port (203) is provided with a regulating mechanism (204) capable of adjusting the opening size thereof.

5. The negative pressure suction sheath according to claim 4, wherein the regulating mechanism (204) is a valve or a slider, the valve or the slider is movably disposed on the pressure regulating port (203), and the opening size of the pressure regulating port (203) is adjusted by pushing the valve or the slider.

6. The negative pressure suction sheath according to claim 1, wherein the proximal end of the main channel cavity (201) is further provided with a sealing washer (4) and a fixing member, and the sealing washer (4) is hermetically fixed at the proximal end of the main channel cavity (201) by using the fixing member; the sealing washer (4) is provided with an inner hole, so that the dilator (3) or/and a surgical device inserted into the main channel cavity (201) is fixedly sleeved in the inner hole of the sealing washer (4) to achieve a fixed connection with the main channel cavity (201).

7. The negative pressure suction sheath according to claim 6, wherein in a natural state, a diameter of the inner hole of the sealing washer (4) is not larger than an outer diameter of the dilator (3) or/and the surgical device.

8. The negative pressure suction sheath according to claim 6, wherein in a natural state, a diameter of the inner hole of the sealing washer (4) is larger than an outer diameter of the dilator (3) or/and the surgical device, a degree of compression on the sealing washer (4) is adjusted by using the fixing member, and then a size of the inner hole of the sealing washer (4) is adjusted, so that the dilator (3) or/and the surgical device inserted into the main channel cavity (201) is fixedly sleeved in the inner hole of the sealing washer (4).

9. The negative pressure suction sheath according to any one of claims 6 to 8, wherein the fixing member is a threaded fixing member (5), the threaded fixing member (5) fixes the sealing washer (4) at the proximal end of the main channel cavity (201) through a threaded connection with the main channel cavity (201), and the degree of compression on the sealing washer (4) may be adjusted by loosening or tightening the threaded fixing member (5).

10. The negative pressure suction sheath according to claim 6, wherein the sheath (1) is a single-cavity or multi-cavity structure, and correspondingly, the sealing washer (4) has one or more inner holes.

11. The negative pressure suction sheath according to claim 6, wherein the surgical device comprises an endoscope (6).

12. The negative pressure suction sheath according to claim 1, wherein a tip end (101) of the sheath (1) is a flexible structure.

13. The negative pressure suction sheath according to claim 1, wherein the sheath (1) is a single-layer tube or a three-layer reinforced tube, if the sheath (1) is a single-layer tube, the sheath (1) is made of a polymer material; or if the sheath (1) is a three-layer reinforced tube, an exterior layer thereof is made of a thermoplastic material, a middle layer thereof is a coiled spring structure or a metal braided wire structure, and an inner layer thereof is a polymer tube with a low friction coefficient.
